(19) Europäisches Patentamt
European Patent Office
Office européen des brevets

(11) Veröffentlichungsnummer: **0 166 695 B1**

# (12) EUROPÄISCHE PATENTSCHRIFT

(45) Veröffentlichungstag der Patentschrift: **18.10.89**

(51) Int. Cl.⁴: **C 07 D 303/34**, C 07 D 301/27

(21) Anmeldenummer: **85810291.6**

(22) Anmeldetag: **24.06.85**

(54) **Verfahren zur Herstellung von Glycidythioethern.**

(30) Priorität: **29.06.84 CH 3148/84**

(43) Veröffentlichungstag der Anmeldung: **02.01.86 Patentblatt 86/1**

(45) Bekanntmachung des Hinweises auf die Patenterteilung: **18.10.89 Patentblatt 89/42**

(84) Benannte Vertragsstaaten: **BE DE FR GB IT**

(56) Entgegenhaltungen:
**EP-A- 0 028 810**
**GB-A- 1 352 527**
**GB-A- 1 359 289**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Wirth, Hermann O., Dr., Lessingstrasse 24, D-6140 Bensheim 3 (DE)**
Erfinder: **Friedrich, Hans-Helmut, Am Rauhenstein 8, D-6147 Lautertal 2 (DE)**

**EP 0 166 695 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betriff ein Verfahren zur Herstellung von Glycidylthioethern.

Glycidylthioether sind Verbindungen, die in der Synthese von organischen Schwefelverbindungen eine wichtige Rolle spielen, die beispielsweise als Schmierstoffadditive Verwendung finden.

Die Herstellung von Glycidylthioethern ist an sich bekannt und in US-PS 2 965 652, US-PS 2 731 437, sowie in CA-PS 909 793 beschrieben. Diese Verfahren verwenden Epichlorhydrin im Überschuss sowie organische Lösungsmittel, was in bezug auf gewerbehygienische und sicherheitstechnische Aspekte nicht unbedenklich ist. Ferner ist aus GB-PS 1 352 527 und GB-PS 1 359 289 ein Verfahren bekannt, das ohne Epichlorhydrinüberschuss arbeitet, jedoch nur die Herstellung von S-Glycidyl-Verbindungen des Cyclododecans bzw. Cyclooctans beschreibt und wobei die angegebenen Ausbeuten auf diese Verbindungen beschränkt sind. Es wurde nun ein Verfahren gefunden, das diese Risikofaktoren nicht aufweist und zudem hohe Ausbeuten an Glycidylthioethern ergibt.

Gegenstand der vorliegenden Erfindung ist ein Verfahren zur Herstellung von Glycidylthioethern der Formel I

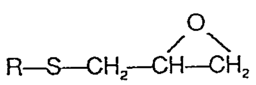 I

durch Umsetzung von äquimalaren Mengen von Epichlorhydrin mit einem Mercaptan der Formel II

R-SH (II), dadurch gekennzeichnet, dass

R ein Rest

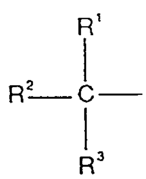

ist, worin $R^1$, $R^2$ und $R^3$ unabhängig voneinander $C_1-C_{18}$ Alkyl sind und zusammen nicht mehr als 22 C-Atome besitzen und $R^2$ und $R^3$ ausserdem Wasserstoff sind, oder dass R $C_5-C_6$-Cycloalkyl, unsubstituiertes oder durch $C_1-C_4$-Alkyl substituiertes Phenyl oder Naphthyl, Benzyl, Furfuryl ist, oder dass R Mercaptoalkyl mit 2 bis 8 C-Atomen bedeutet, das gegebenenfalls durch 1 oder 2 Ether-O-Atome unterbrochen sein kann, und dass die Umsetzung ohne organisches Lösungsmittel in Gegenwart einer Base, nach dem Phasentransfer-Katalyse-Verfahren, in Kontakt mit einer wässrigen Phase, wobei ein Phasentransfer-Katalysator eingesetzt wird, bei einer Temperatur von -30 °C bis +20 °C ausgeführt wird.

Stellt R ein Radikal der Form

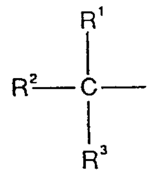

dar, so kann es sich um $R^1-CH_2-$,

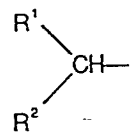

oder um

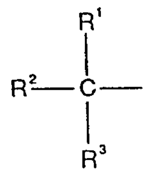

handeln, wobei $R^1$, $R^2$ und $R^3$ jeweils $C_1-C_{18}$-Alkyl, sind. Bei $C_1-C_{18}$-Alkyl handelt es sich um geradkettige oder verzweigte Substituenten, wie beispielsweise Methyl, Ethyl, n-Propyl, Isopropyl, n-Butyl, Isobutyl, sec.-Butyl, tert.-Butyl, geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl. Bevorzugt ist

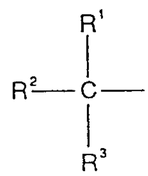

worin $R^1$, $R^2$ und $R^3$ zusammen mit dem C-Atom, an das sie gebunden sind, $C_4-C_{20}$-Alkyl bilden, wobei keiner dieser Substituenten $R^1$, $R^2$ und $R^3$ Wasserstoff sein darf; besonders bevorzugt ist hier nun $C_4-C_{16}$-Alkyl, insbesondere ist tert.-Butyl, tert.-Nonyl (ex Phillips Petroleum) oder tert.-Dodecyl bevorzugt, wobei z. B. unter tert.-Dodecyl solch ein Rest verstanden werden soll, wie er für tertiäres Dodecylmercaptan in «Ulmanns Enzyklopädie der technischen Chemie, 4. Auflage, Band 23, Seite 181-182, Verlag Chemie, Weinheim» beschrieben ist.

Stellt R $C_5-C_6$-Cycloalkyl dar, so handelt es sich um Cyclopentyl oder Cyclohexyl, vorzugsweise Cyclohexyl.

Stellt R durch $C_1-C_4$-Alkyl substituiertes Phenyl oder Naphtyhl dar, so können Phenyl oder Naphthyl ein- bis dreifach, bevorzugt jedoch einfach, substituiert sein; bei $C_1-C_4$-Alkyl handelt es sich um Methyl, Ethyl, n-Propyl, Isopropyl, n-Butyl, Isobutyl, sec.-Butyl oder tert.-Butyl.

Stellt R Mercaptoalkyl mit 2 bis 8 C-Atomen dar, das gegebenenfalls durch 1 oder 2 Ether-O-Atome unterbrochen ist, so handelt es sich um einfach substituiertes $C_2-C_8$ Mercaptoalkyl, wobei die Substitution durch die SH-Gruppe in jeder Position möglich, vorzugsweise aber terminal ist, insbesondere handelt es sich um $-CH_2-CH_2-SH$.

Bevorzugt werden nach dem erfindungsgemässen Verfahren Glycidylthioether der Formel I aus Epichlorhydrin und einem Mercaptan der Formel II hergestellt, worin R ein Radikal der Form

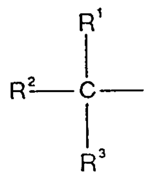

ist, worin $R^1$, $R^2$ und $R^3$ unabhängig voneinander $C_1-C_{18}$ Alkyl sind und zusammen nicht mehr als 22 C-Atome besitzen und $R^2$ und $R^3$ ausserdem Wasserstoff sind, oder worin R Mercaptoalkyl mit 2 bis 8 C-Atomen ist, das gegebenenfalls durch 1 oder 2 Ether-O-Atome unterbrochen ist und die $-SH$-Gruppe terminal gebunden ist.

Besonders bevorzugt werden nach dem erfindungsgemässen Verfahren Glycidylthioether der Formel I durch Umsetzung von Epichlorhydrin mit einem Mercaptan der Formel II hergestellt, worin R $-CH_2CH_2-SH$ oder ein Radikal der Form

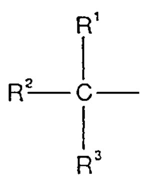

ist, worin $R^1$, $R^2$ und $R^3$ zusammen mit dem C-Atom, an das sie gebunden sind, $C_4-C_{20}$ Alkyl bilden, wobei keiner dieser Substituenten $R^1$, $R^2$ und $R^3$ Wasserstoff sein darf.

Das erfindungsgemässe Verfahren wird in Gegenwart einer Base durchgeführt, die in bezug auf den Reaktionspartner Mercaptan in aequimolarer Menge oder leichtem Überschuss eingesetzt wird. Geeignete Basen sind z.B. Alkalihydroxide wie Natrium-, Kalium- oder Lithiumhydroxid oder Erdalkalihydroxide wie Calcium- oder Magnesiumhydroxid. Bevorzugt verwendet man als Base Alkalihydroxide insbesondere Natriumhydroxid.

Das erfindungsgemässe Verfahren wird in Gegenwart eines Phasentransferkatalysators durchgeführt, der in üblicher Menge, z.B. mindestens 0,6 Gew.-% bezogen auf die eingesetzte Menge Mercaptan und Epichlorhydrin eingesetzt wird.

Als Phasentransferkatalysatoren eignen sich verschiedene, wie sie z.B. in CHEMTECH, Februar 1980, S. III, Tabelle 1 aufgeführt sind, z.B. quaternäre Salze, Polyether, N-Alkylphosphoramide.

Bevorzugt werden im erfindungsgemässen Verfahren quaternäre Ammoniumsalze der Formel III

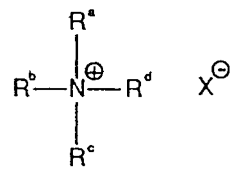

III

worin $R^a$, $R^b$, $R^c$ und $R^d$ gleich oder verschieden sind und geradkettige oder verzweigte Alkylreste, wobei alle 4 Alkylreste zusammen 4 bis 20 C-Atome besitzen, oder Benzyl bedeuten und $X^\ominus$, $Br^\ominus$, $Cl^\ominus$, $HSO_4^\ominus$ oder $CH_3SO_4^\ominus$ darstellt, eingesetzt.

$R^a$, $R^b$, $R^c$ und $R^d$ können folgende Alkylreste bedeuten: z.B. Methyl, iso-Propyl, n-Butyl, sec.-Butyl, tert.-Butyl, tert.-Amyl, 2-Ethylhexyl, n-Octyl, 1,1,3,3-Tetramethylbutyl, n-Dodecyl.

Besonders bevorzugt werden als Phasentransferkatalysator $(n-C_4H_9)_4NCl$ und $(n-C_4H_9)_4NBr$, insbesondere $(n-C_4H_9)_4NBr$, eingesetzt.

Die Umsetzung der Reaktionspartner im erfindungsgemässen Verfahren erfolgt bei einer Temperatur von -30°C bis 20°C, vorzugsweise bei einer Temperatur von 10-20°C, wobei die Maximaltemperatur nicht überschritten werden soll.

Die Phasentrennung erfolgt für viskose Reaktionsprodukte vorzugsweise bei erhöhter Temperatur.

Eine bevorzugte Ausführungsform des erfindungsgemässen Verfahrens besteht darin, dass das Mercaptan der Formel II, Epichlorhydrin sowie der Phasentransferkatalysator vorgelegt werden und bei einer Temperatur von weniger als 20°C eine katalytische Menge einer wässrigen Lösung der Base zugegeben wird und anschliessend die Base, vermindert um die katalytische Menge, zugegeben wird.

Besonders bevorzugt wird das erfindungsgemässe Verfahren in der Ausführungsform, worin das Mercaptan der Formel II und der Phasentransferkatalysator vorgelegt werden und bei einer Temperatur von weniger als 20°C Epichlorhydrin und eine katalytische Menge einer wässrigen Lösung der Base zugegeben werden und anschliessend die Base, vermindert um die katalytische Menge, zugegeben wird.

Vorzugsweise werden Epichlorhydrin und die katalytische Menge an Base getrennt aber gleichzeitig zugegeben.

Die katalytische Menge an Base beträgt ungefähr 2 bis 5% der Gesamt-Menge an Base.

Es ist möglich, das erfindungsgemässe Verfahren zu automatisieren, z.B. die Epichlorhydrin-Zugabe durch Temperaturmessung und die Basen-Zugabe durch pH-Messung.

Beispiele für die nach dem erfindungsgemässen Verfahren herstellbaren Glycidylthioether der Formel I sind folgende:
tert.-Octylglycidylthioether
n-Octylglycidylthioether

tert.-Nonylglycidylthioether
tert.-Dodecylglycidylthioether
n-Dodecylglycidylthioether
tert.-Hexadecylglycidylthioether
Cyclohexylglycidylthioether
α-Naphthylglycidylthioether
Benzylglycidylthioether
Furfurylglycidylthioether
Ethylen-diglycidylthioether, hergestellt durch Umsetzung von 2 Molen Epichlorhydrin mit einem Mol Dimercaptoethan nach dem erfindungsgemässen Verfahren, demnach dass die Umsetzung ohne organisches Lösungsmittel in Gegenwart einer Base, nach dem Phasentransferkatalyse-Verfahren, in Kontakt mit einer wässrigen Phase, wobei ein Phasentransferkatalysator eingesetzt wird, bei einer Temperatur von −30°C bis +20°C ausgeführt wird.

Beispiel 1: tert-Octylglycidylthioether

Zu einer Mischung aus 219 Gewichtsteilen tert-Octylmercaptan und 135 Gewichtsteilen Epichlorhydrin wird bei 15 bis 20°C unter Rühren und teilweiser Kühlung (besonders am Anfang der Zugabe) eine Lösung aus 66 Gewichtsteilen Natriumhydroxid, 300 Gewichtsteilen Wasser und 8 Gewichtsteilen Tetrabutylammoniumchlorid innerhalb von 70 Minuten zugetropft. Das Reaktionsgemisch wird 1 Stunde bei 50°C nachgerührt, die wässrige Phase wird abgetrennt, und die organische Phase wird mit 200 Gewichtsteilen Wasser gewaschen. Schliesslich wird dann die organische Phase im Vakuum destilliert, und man erhält den tert-Octylglycidylthioether als farblose Flüssigkeit mit einem Siedepunkt von 74 bis 75°C bei 0,02 Torr und einem Brechungsindex von $n_D^{20}$ = 1,4803; die Ausbeute beträgt 250 Gewichtsteile, was 82% der theoretischen Ausbeute entspricht.

Beispiel 2: tert-Nonylglycidylthioäther

In einem 1 l Sovirel-Reaktor (doppelwandiges Reaktionsgefäss) mit Thermometer, Rührer, Dosiertropftrichter und Rückflusskühler wird 321 g tert-Nonylmercaptan, 184 g Epichlorhydrin und 4 g Tetrabutylammoniumchlorid vorgelegt. Zu dieser Lösung wird unter Rühren und intensiver Wasserkühlung 30 ml einer 22%igen wässrigen Natriumhydroxidlösung innerhalb 45 Minuten, und nach Abklingen der exothermen Reaktion nochmals 285 ml 22%ige Natriumhydroxidlösung innerhalb 60 Minuten, bei 15–20°C zugetropft. Das Reaktionsgemisch wird 30 Minuten bei 50°C nachgerührt, die wässrige Phase abgetrennt, die organische Phase 2 mal mit je 200 g Wasser gewaschen und die organische Phase im Vakuum getrocknet. Rückstand: 422 g (ber. 433 g) $n_D^{20}$ : 1,4813; lt. NMR enthält der Rückstand 92 Gew.-% tert-Nonylglycidylthioäther.

Beispiel 3: n-Octylglycidylthioäther

In einem 1 l Sovirel-Reaktor (doppelwandiges Reaktionsgefäss) mit Thermometer, Rührer, Tropftrichter, Dosiertropftrichter und pH-Elektrode wird 293 g n-Octylmercaptan und 5 g Tetrabutylammoniumchlorid vorgelegt. Zu dieser Lösung wird unter intensiver Wasserkühlung und Rühren 184 g Epichlorhydrin innerhalb 80 Minuten bei einem pH-Wert von 11,6–12,0 eingetropft. Der pH-Wert wird durch entsprechende Dosierung von ca. 10 ml 22%iger wässriger Natriumhydroxidlösung über den gesamten Zeitraum von 80 Minuten konstant gehalten. Die Reaktionstemperatur sollte 20°C nicht übersteigen. Nach beendeter exothermer Reaktion wird 305 ml 22%ige Natriumhydroxidlösung innerhalb 15 Minuten eingetropft, das Reaktionsgemisch 30 Minuten bei 50°C nachgerührt, die wässrige Phase abgetrennt, die organische Phase 2 mal mit je 200 g Wasser gewaschen und die organische Phase im Vakuum fraktioniert. Die Ausbeute beträgt 382 g (94% d.Th.) mit einem Siedepunkt von 92–94°C bei 0,04 Torr mit einem Brechungsindex von $n_D^{20}$ : 1,4718.

Die folgenden Glycidylthioether wurden analog zu den in den Beispielen 2 und 3 beschriebenen Verfahren hergestellt.

| Formel | Analyt. Dat. ($n_D^{20}$; Smp.) | Hergestellt nach allg. Beispiel | Bemerkungen Ausbeute |
|---|---|---|---|
| 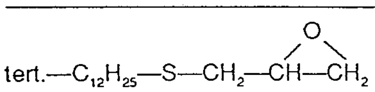 | 98–110°/0.05 $n_D^{20}$: 1.4790 | 2 | 86 Gew.-%2) |
| 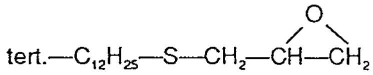 | | 3 | 90 Gew.-%1) |
| 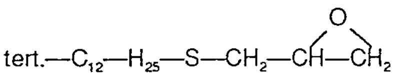 | 125–28°/0.06 $n_D^{20}$: 1.4717 | 3 | 99 Gew.-%1) |
| 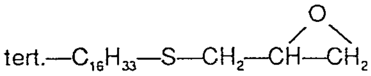 | 118–20°/0.06 $n_D^{20}$: 1.4844 | 2 | 93 Gew.-%1) |

| Formel | Analyt. Dat. ($n_D^{20}$; Smp.) | Hergestellt nach allg. Beispiel | Bemerkungen Ausbeute |
|---|---|---|---|
| 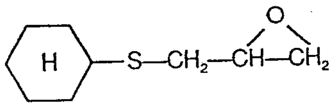 | 114-16°/0.8 $n_D^{20}$: 1.5074 | 3 | 95 Gew.-%[1)] |
| 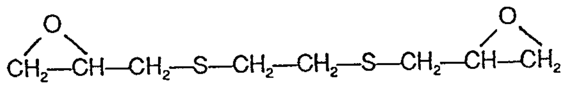 | $n_D^{20}$: 1.5473 | 3 | 94 Gew.-%[3)] |

1) Bestimmung des Epoxidgehaltes im Rohprodukt via NMR
2) Nach Destillation
3) Bestimmung des Epoxidgehaltes im Rohprodukt via potentiometr. Titration

## Patentansprüche

1. Verfahren zur Herstellung von Glycidylthioethern der Formel I

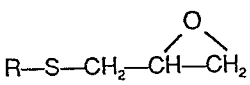 I

durch Umsetzung von aequimolaren Mengen von Epichlorhydrin mit einem Mercaptan der Formel II

R-SH (II),

dadurch gekennzeichnet, dass

R ein Rest

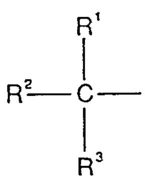

ist, worin $R^1$, $R^2$ und $R^3$ unabhängig voneinander $C_1-C_{18}$ Alkyl sind und zusammen nicht mehr als 22 C-Atome besitzen und $R^2$ und $R^3$ ausserdem Wasserstoff sind, oder dass R $C_5-C_6$ Cycloalkyl, unsubstituiertes oder durch $C_1-C_4$ Alkyl substituiertes Phenyl oder Naphthyl, Benzyl, Furfuryl ist, oder dass R Mercaptoalkyl mit 2 bis 8 C-Atomen bedeutet, das gegebenenfalls durch 1 oder 2 Ether-O-Atome unterbrochen sein kann, und dass die Umsetzung ohne organisches Lösungsmittel in Gegenwart einer Base, nach dem Phasentransferkatalyseverfahren, in Kontakt mit einer wässrigen Phase, wobei ein Phasentransferkatalysator eingesetzt wird, bei einer Temperatur von -30°C bis +20°C ausgeführt wird.

2. Verfahren gemäss Anspruch 1, dadurch gekennzeichnet, dass R ein Radikal der Form

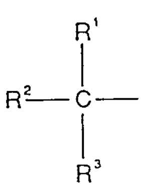 ist,

worin $R^1$, $R^2$ und $R^3$ unabhängig voneinander $C_1-C_{18}$ Alkyl sind und zusammen nicht mehr als 22 C-Atome besitzen und $R^2$ und $R^3$ ausserdem Wasserstoff sind, oder worin R Mercaptoalkyl mit 2 bis 8 C-Atomen ist, das gegebenenfalls durch 1 oder 2 Ether-O-Atome unterbrochen ist und die -SH-Gruppe terminal gebunden ist.

3. Verfahren gemäss Anspruch 1, dadurch gekennzeichnet, dass R $-CH_2CH_2-SH$ oder ein Radikal der Form

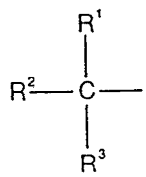

ist, worin $R^1$, $R^2$ und $R^3$ zusammen mit dem C-Atom, an das sie gebunden sind, $C_4-C_{20}$ Alkyl bilden, wobei keiner dieser Substituenten $R^1$, $R^2$ und $R^3$ Wasserstoff sein darf.

4. Verfahren gemäss Anspruch 1, dadurch gekennzeichnet, dass als Base ein Alkalihydroxid oder ein Erdalkalihydroxid eingesetzt wird.

5. Verfahren gemäss Anspruch1, dadurch gekennzeichnet, dass der Phasentransferkatalysator ein quaternäres Ammoniumsalz der Formel III

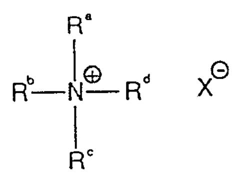 III

ist, worin $R^a$, $R^b$, $R^c$ und $R^d$ gleich oder verschieden sind und geradkettige oder verzweigte Alkylreste, wobei alle 4 Alkylreste zusammen 4 bis 20 C-Atome besitzen, oder Benzyl bedeuten und $X^{\ominus}$, $Br^{\ominus}$, $Cl^{\ominus}$, $HSO_4^{\ominus}$ oder $CH_3SO_4^{\ominus}$ darstellt.

6. Verfahren gemäss Anspruch 1, dadurch gekennzeichnet, dass der Phasentransferkatalysator in einer Menge von mindestens 0.6 Gew.-%, bezogen auf die eingesetzte Menge Mercaptan und Epichlorhydrin, eingesetzt wird.

7. Verfahren gemäss Anspruch1, dadurch gekennzeichnet, dass das Mercaptan der Formel II, Epichlorhydrin sowie der Phasentransferkatalysator vorgelegt werden und bei einer Temperatur von weniger als 20°C eine katalytische Menge einer wässrigen Lösung der Base zugegeben wird und anschliessend die Base, vermindert um die katalytische Menge, zugegeben wird.

8. Verfahren gemäss Anspruch 1, dadurch gekennzeichnet, dass das Mercaptan der Formel II und der Phasentransferkatalysator vorgelegt werden und bei einer Temperatur von weniger als 20°C Epichlorhydrin und eine katalytische Menge einer wässrigen Lösung der Base zugegeben werden und anschliessend die Base, vermindert um die katalytische Menge, zugegeben wird.

9. Verfahren zur Herstellung von Ethylendiglycidylthioether durch Umsetzung von 2 Molen Epichlorhydrin mit einem Mol Dimercaptoethan, dadurch gekennzeichnet, dass die Umsetzung ohne organisches Lösungsmittel in Gegenwart einer Base, nach dem Phasentransferkatalyseverfahren, in Kontakt mit einer wässrigen Phase, wobei ein Phasentransferkatalysator eingesetzt wird, bei einer Temperatur von -30°C bis +20°C ausgeführt wird.

## Claims

1. A process for producing glycidyl thioethers of the formula I

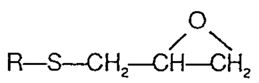

I

by reacting equimolar amounts of epichlorohydrin with a mercaptan of the formula II

R−SH (II)

wherein R is a radical

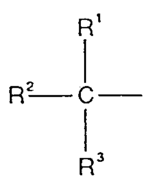

in which $R^1$, $R^2$ and $R^3$ independently of one another are each $C_1-C_{18}$alkyl and together have no more than 22 C atoms, and $R^2$ and $R^3$ are in addition hydrogen, or wherein R is $C_5-C_6$cycloalkyl, phenyl or naphthyl unsubstituted or substituted by $C_1-C_4$alkyl, or is benzyl or furfuryl, or wherein R is mercaptoalkyl which has 2 to 8 C atoms and can be interrupted by 1 or 2 ether-O atoms, and wherein the reaction is carried out at a temperature of -30°C to +20°C without organic solvent and in the presence of a base, by the phase-transfer catalysis process, in contact with an aqueous phase, a phase-transfer catalyst being employed.

2. A process according to claim 1, wherein R ist a radical of the form

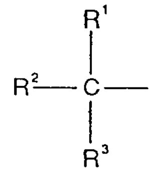

in which $R^1$, $R^2$ and $R^3$ independently of one another are each $C_1-C_{18}$alkyl, and together have not more than 22 C atoms, and $R^2$ and $R^3$ are in addition hydrogen, or in which R is mercaptoalkyl which has 2 to 8 C atoms and can be interrupted by 1 or 2 ether-O atoms, and the −SH group is bound in the terminal position.

3. A process according to claim 1, wherein R is $-CH_2-CH_2-SH$ or a radical of the form

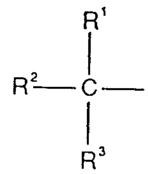

in which $R^1$, $R^2$ and $R^3$ together with the C atom to which they are bound form $C_4-C_{20}$alkyl, where none of these substituents $R^1$, $R^2$ and $R^3$ may be hydrogen.

4. A process according to claim 1, wherein the base used is an alkali metal hydroxide or an alkaline earth metal hydroxide.

5. A process according to claim 1, wherein the phase-transfer catalyst is a quaternary ammonium salt of the formula III

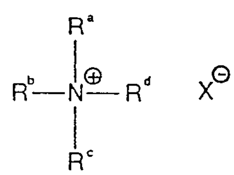

III

in which $R^a$, $R^b$, $R^c$ and $R^d$ are identical or different, and are straight-chain or branched-chain alkyl radicals, all 4 alkyl radicals together having 4 to 20 C atoms, or are benzyl, and $X^\ominus$ is $Br^\ominus$, $Cl^\ominus$, $HSO_4^\ominus$ or $CH_3SO_4^\ominus$.

6. A process according to claim 1, wherein the phase-transfer catalyst is used in an amount of at least 0.6% by weight, relative to the employed amount of mercaptan and epichlorohydrin.

7. A process according to claim 1, wherein the mercaptan of the formula II, epichlorohydrin and the phase-transfer catalyst are placed into the reaction vessel and, at a temperature of less than 20°C, a catalytic remainder of the base is added.

8. A process according to claim 1, wherein the mercaptan of the formula II and the phase-transfer catalyst are placed into the reaction vessel and, at a temperature of less than 20°C, epichlorohydrin and a catalytic amount of an aqueous solution of the base are added, and subsequently the remainder of the base is added.

9. A process for producing ethylenediglycidyl thioethers by reacting 2 moles of epichlorohydrin

with 1 mole of dimercaptoethane, wherein the reaction is carried out at a temperature of -30°C to +20°C without organic solvent and in the presence of a base, by the phase-transfer catalysis process, in contact with an aqueous phase, a phase-transfer catalyst being employed.

**Revendications**

1. Procédé pour préparer des thio-éthers glycidyliques répondant à la formule I:

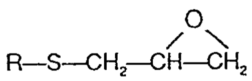

I

par réaction de quantités équimolaires d'épichlorhydrine avec un mercaptan de formule II:

R−SH (II),

procédé caractérisé en ce que R est un radical:

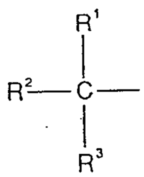

dans lequel $R^1$, $R^2$ et $R^3$ représentent chacun, indépendamment les uns des autres, un alkyle en $C_1-C_{18}$ et, ensemble, ne contiennent pas plus de 22 atomes de carbone, et $R^2$ et $R^3$ peuvent en outre représenter chacun l'hydrogène, ou en ce que R représente un cycloalkyle en $C_5$ ou $C_6$, un radical phényle ou napthyle non substitué ou porteur d'un alkyle en $C_1-C_4$, un benzyle ou un furfuryle, ou en ce que R représente un mercapto-alkyle contenant de 2 à 8 atomes de carbone et éventuellement interrompu par un ou deux atomes d'oxygène de fonction éther, et en ce que la réaction est executée à une température de -30°C à +20°C sans solvant organique, en présence d'une base, par la méthode de catalyse avec transfert de phases, au contact d'une phase aqueuse, en présence d'un catalyseur de transfert de phases.

2. Procédé selon la revendication 1 caractérisé en ce que R représente un radical de formule:

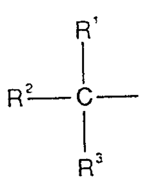

dans laquelle $R^1$, $R^2$ et $R^3$ représentent chacun, indépendamment les uns des autres, un alkyle en $C_1-C_{18}$ et, ensemble, ne contiennent pas plus de 22 atomes de carbone, et $R^2$ et $R^3$ peuvent en outre représenter chacun l'hydrogène, ou R représente un mercapto-alkyle en $C_2-C_8$ qui est éventuellement interrompu par un ou deux atomes d'oxygène de fonction éther et qui porte le radical −SH en position terminale.

3. Procédé selon la revendication 1 caractérisé en ce que R représente un radical $-CH_2-CH_2-SH$ ou un radical répondant à la formule:

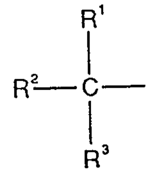

dans laquelle $R^1$, $R^2$ et $R^3$, avec l'atome de carbone auquel ils sont liés, forment un alkyle en $C_4-C_{20}$, aucun de ces symboles $R^1$, $R^2$ et $R^3$ ne représentant l'hydrogène.

4. Procédé selon la revendication 1 caractérisé en ce qu'on utilise, comme base, un hydroxyde de métal alcalin ou un hydroxyde de métal alcalino-terreux.

5. Procédé selon la revendication 1 caractérisé en ce que le catalyseur de transfert de phases est un sel d'ammonium quaternaire répondant à la formule III:

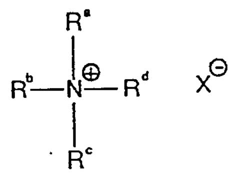

III

dans laquelle $R^a$, $R^b$, $R^c$ et $R^d$ représentent chacun, indépendamment les uns des autres, un radical alkyle linéaire ou ramifié, ces quatre radicaux alkyles contenant ensemble de 4 à 20 atomes de carbone, ou un radical benzyle, et $X^\ominus$ représente $Br^\ominus$, $Cl^\ominus$, $HSO_4^\ominus$ ou $CH_3SO_4^\ominus$.

6. Procédé selon la revendication 1 caractérisé en ce que le catalyseur de transfert de phases est mise en jeu en une quantité d'au moins 0,6% enpoids par rapport à la quantité utilisée de mercaptan et d'éichlorhydrine.

7. Procédé selon la revendiction 1 caractérisé en ce qu'on met dès le départ dans le récipient le mercaptan de formule II, l'épichlorhydrine et le catalyseur de transfert de phases, puis on ajoute, à une température inférieure à 20°C, une quantité catalytique d'une solution aqueuse de la base, après quoi on ajoute la base en une quantité diminuée de la quantité catalytique.

8. Procédé selon la revendication 1 caractérisé en ce qu'on place dès le départ dans le récipient le mercaptan de formule II et le catalyseur de transfert de phases, puis on ajoute, à une température inférieure à 20°C, l'épichlorhydrine et une quantité catalytique d'une solution aqueuse de la base, après quoi on ajoute la base en une quantité diminuée de la quantité catalytique.

9. Procédé de préparation du bis-(glycidylthio)-1,2 éthane par réaction de 2 mol d'épichlorhydrine avec 1 mol de dimercapto-éthane, procédé caractérisé en ce qu'on effectue la réaction à une température de -30°C à +20°C, sans solvant organique, en présence d'une base, par la méthode de catalyse avec transfert de phases, au contact d'une phase aqueuse et en présence d'un catalyseur de transfert de phases.